## Europäisches Patentamt

(19) **European Patent Office**

**Office européen des brevets**

(11) Publication number: **0 206 240 B1**

(12) **EUROPEAN PATENT SPECIFICATION**

(45) Date of publication of patent specification: **05.09.90**

(51) Int. Cl.⁵: **A 61 K 9/20**, A 61 K 9/30, A 61 K 31/205, A 61 K 31/685

(21) Application number: **86108309.5**

(22) Date of filing: **18.06.86**

(54) Solid pharmaceutical composition for oral use.

(30) Priority: **19.06.85 IT 2121485**

(43) Date of publication of application:
**30.12.86 Bulletin 86/52**

(45) Publication of the grant of the patent:
**05.09.90 Bulletin 90/36**

(84) Designated Contracting States:
**AT BE CH DE FR GB IT LI LU NL SE**

(56) References cited:
EP-A-0 209 037
GB-A-2 021 414
GB-A-2 052 976
US-A-3 830 931

(73) Proprietor: **ZAMBON S.p.A.**
**Via della Chimica, 9**
**I-36100 Vicenza (IT)**

(72) Inventor: **Gazzaniga, Annibale, Dr.**
**Via Generale Porro, 22**
**I-20027 Rescaldina (Milano) (IT)**
Inventor: **Stroppolo, Federico, Dr.**
**Via Vedreggio, 17**
**CH-6963 Pregassona (CH)**
Inventor: **Macciocchi, Alberto, Dr.**
**Via alla Bola, 2**
**CH-6815 Melide (CH)**

(74) Representative: **Weinhold, Peter, Dr. et al**
**Patentanwälte Dipl.-Ing. G. Dannenberg Dr. P.**
**Weinhold Dr. D. Gudel Dipl.-Ing. S. Schubert Dr.**
**P. Barz Siegfriedstrasse 8**
**D-8000 München 40 (DE)**

Courier Press, Leamington Spa, England.

# EP 0 206 240 B1

**Description**

The present invention relates to a solid pharmaceutical preparation and more particularly relates to a solid pharmaceutical preparation for oral use containing carnitine and lecithin. L-Carnitine is a natural constituent of striated muscle and liver and is used in therapeutic field as an antihyperlipoproteinemic agent, in Duchenne and Beker's muscular dystrophia, in cases of general muscular deficiency, in cases of mycardium ischemia, in such pathologies of liver and spleen and as a prompt source of energy for rapid utilization. L-Carnitine is an extremely hygroscopic and deliquescent, very soluble white powder, with a characteristic saline and anesthetic taste. A similar behaviour has been found as to D,L-carnitine, L-acetylcarnitine, D,L-acetylcarnitine and other acyl derivatives. In the present invention with the term carnitine we intend to refer to L-carnitine as well as to D,L-carnitine and to the corresponding acyl derivatives or to pharmaceutically acceptable salts.

Lecithin is a natural phospholipid from seeds of clovers such as soybean, or from egg yolk, and it is used in the therapeutic field as a lipotropic agent. Lecithin is an oil or a sticky greasy powder, with a characteristic taste and smell, easily oxidizable (going rancid) by UV radiation as well as by atmospheric oxygen. The contemporaneous administration of carnitine and lecithin does not show a synergistic effect between the substances which instead perform their specific actions separately.

Nevertheless, their action may be considered as complementary since carnitine is a corroborant for the muscular system and lecithin is a corroborant for the nervous system.

The association of carnitine and lecithin, for its character of nervous-muscular corroborant, appears to be suitable for the treatment where there is an increased need of muscular energy, or in cases of psychophysical asthenia, of debilitating chronic diseases and in the periods of convalescence.

The Swiss Patent No. 637,295 in the name of Claudio Cavazza describes a liquid composition for parenteral administration containing triglycerides (for instance egg-lecithin) and carnitine and suitable for the treatment of patients in state of shock or which have undergone a trauma.

In the case of liquid compositions intended for parenteral or intraveous route, such as, for instance, the compositions described in the above Swiss Patent, the administration of the drug is often a long complex operation that generally cannot be performed by the patient himself but only in hospitals.

Thus, whenever possible, it is preferred to have available an orally ingestible solid pharmaceutical preparation (for instance a tablet or a sugar coated tablet) because the patient can take the drug directly at pre-established doses and intervals.

Carnitine is a hygroscopic deliquescent substance while lecithin is hygroscopic and easily oxidable in the light.

These unfavourable characteristics essentially prevent the use of solid pharmaceutical preparations of the traditional type which contain such an association because carnitine causes, in very short time, phenomena of physical degradation of the solid pharmaceutical preparations (swelling and disintegration) and lecithin, in contact with air and light, goes rancid making the preparations unsuitable for consumption.

The problems of physical stability and of conservation of these traditional solid pharmaceutical preparations become pratically insurmountable in the case of retail sales by the pharmacist as this inevitably involves storage periods of the packed drug.

We have now found that by thorough mixing of carnitine and lecithin with a substance selected among shellac, rosin, turpentine, cellulose acetate phthalate, polyacrylamide resin or mixtures thereof, and usual excipients and additives, it is possible to prepare solid pharmaceutical preparations, for instance chewable tablets, which are physically stable for a long period of time and in which the association carnitine-lecithin remains unaltered for a period of three years or more under usual storage conditions.

Therefore, object of the present invention are solid pharmaceutical preparations for oral use consisting of carnitine, lecithin and usual excipients and additives and characterized in that they also contain an amount, comprised between 1 and 60% weight by weight (preferably between 3 and 5% w/w) with respect to carnitine plus lecithin, of a substance selected among shellac, rosin, turpentine, cellulose acetate phthalate, polyacrylamide resin or mixtures thereof.

The above solid pharmaceutical preparations in the form of chewable tablts are a preferred embodiment of the invention.

For the sake of brevity the substances characterizing the invention, that is shellac, rosin, turpentine, cellulose acetate, phthalate, polyacrylamide resin or mixtures thereof, will be indicated below by the term "stabilizers".

The term "stabilizer" related to these compounds in the present application indicates that they are able to physically stabilize the solid pharmaceutical preparations of the present invention.

While on the one hand it is essential that the stabilizing substance be used in a thorough mixture with carnitine, lecithin and the other components, it is also possible to use part of this stabilizer to externally coat the solid pharmaceutical preparation especially when this is not a sugar coated chewable tablet.

The excipients and additives used in the solid pharmaceutical preparations of the invention comprise the usual diluents, binders, disintegrants, lubricants, flavouring agents, sweetening agents and coloring agents used in the preparation of solid pharmaceutical compositions.

In the case of chewable tablets suitable excipients and additives able to impart good characteristics of palatability to the tablets will be selected.

2

Some specific examples of these compounds are, among the diluents, saccharose, sorbitol, lactose and generally mono- or polysaccharides, among the lubricants, alkaline or alkaline-earth stearates, saturated and unsaturated fatty acids and, besides the flavouring agents or auxiliary flavouring agents, such as natural or natural-like flavors, ascorbic acid or citric acid, and sweetening agents like sugars or cyclamates, saccharin and aspartame.

Clearly, also in the case of chewable tablets, it will be necessary to use binders and disintegrants such as starch, variously substituted celluloses, polyethylene glycols, silicon dioxide, polyvinylpyrrolidinone and so on. Then the compositions may contain natural coloring agents such as carotenes and ferric oxide, or pharmaceutically acceptable artificial coloring agents.

The preparation of the pharmaceutical compositions of the invention does not present any particular difficulty. The thorough mixing of carnitine, lecithin, excipients and additives with the stabilizer according to the present invention may be carried out by usual techniques of mixing, compression or granulation of the different components, after which a further coating with the selected stabilizing agent may follow.

This last operation may be carried out by the usual apparatus for film-coating (pan, fluid bed). If desired, the tablets so prepared may then be sugar coated with sugary syrups or with a film of coating and opaquizing substances.

For example a chewable tablet according to the present invention with a weight of 1.5—2 g consists of:

from 5 to 15% w/w of carnitine or a derivative thereof,

from 10 to 40% w/w of lecithin,

from 1 to 5% w/w of stabilizer,

the rest to 100% w/w being constituted by usual pharmaceutically acceptable excipients and additives which comprise, as above reported, diluents, lubricants, binders, disintegrants, flavoring agents and sweetening agents.

If the diluent consists of a sugar (saccharose, lactose, sorbitol and so on) the use of sweetening agents may be unnecessary.

The number of tablets to be administered daily will be decided by the physician also in relation to different factors connected with the condition of the patient.

The daily amount of tablets to be administered will be also in relation to the fact that the daily dose of carnitine is generally comprised between 400 and 2000 mg and the dose of lecithin between 1500 and 6000 mg, however, higher dosages being acceptable according to the individual needs.

In order to better illustrate the invention without limiting it the following examples are now given.

## Example 1
A homogeneous mixture of the following ingredients:

| | |
|---|---|
| L-Carnitine | 150 g |
| Lecithin | 500 g |
| Shellac | 40 g |
| Sorbitol | 550 g |
| Silicon dioxide | 40 g |
| Magnesium stearate | 17 g |
| Saccharose | 100 g |
| Maize starch | 300 g |
| Ascorbic acid | 3 g |

is prepared by mixing L-carnitine, lecithin and shellac.

The mixture is homogenized and pressed, then again milled and sieved to obtain homogeneous particle size. The other ingredients (excipients and additives) are added to the mixture and the whole is mixed aain.

The resulting homogeneous mixture is pressed to a 1.7 g tablet.

## Example 2
A homogeneous mixture of the following ingredients:

| | |
|---|---|
| L-Carnitine | 150 g |
| Lecithin | 500 g |
| Shellac | 40 g |
| Sorbitol | 550 g |
| Silicon dioxide | 40 g |
| Magnesium stearate | 17 g |
| Saccharose | 100 g |
| Maize starch | 300 g |
| Ascorbic acid | 3 g |

is prepared by mixing L-carnitine with lecithin. The mixture is then put into a suitable granulator and it is granulated with a solution of shellac in ethyl alcohol. The obtained granulate is dried and then sieved. The other ingredients are added to it and, after mixing, the whole is pressed to a 1.7 g tablet.

## Example 3
A homogeneous mixture of the following ingredients:

| | |
|---|---|
| L-Carnitine | 150 g |
| Lecithin | 500 g |
| Shellac | 40 g |
| Sorbitol | 550 g |
| Silicon dioxide | 40 g |
| Magnesium stearate | 17 g |
| Saccharose | 100 g |
| Maize starch | 300 g |
| Ascorbic acid | 3 g |

is prepared by collecting the powders in a mixer to homogenize the whole.

The mixture is pressed to a 1.7 g tablet. Further 400 g of shellac are dissolved in ethyl alcohol and the solution is sprayed onto the tablets in a suitable apparatus for film-coating (fluid bed) until obtaining a uniform coating.

### Example 4
A homogeneous mixture of the following ingredients:

| | |
|---|---|
| L-Acetylcarnitine | 150 g |
| Lecithin | 500 g |
| Cellulose acetate phthalate | 40 g |
| Sorbitol | 550 g |
| Silicon dioxide | 40 g |
| Magnesium stearate | 17 g |
| Saccharose | 100 g |
| Maize starch | 300 g |
| Ascorbic acid | 3 g |

is prepared according to the method described in example 1 in order to obtain tablets of 1.7 g each.

### Example 5
A homogeneous mixture of the following ingredients:

| | |
|---|---|
| L-Carnitine | 150 g |
| Lecithin | 500 g |
| Cellulose acetate phthalate | 40 g |
| Sorbitol | 550 g |
| Silicon dioxide | 40 g |
| Magnesium stearate | 17 g |
| Saccharose | 100 g |
| Maize starch | 300 g |
| Ascorbic acid | 3 g |

is prepared by collecting the powders in a mixer to homogenize the whole. The mixture is pressed to a 1.7 g tablet.

The resulting core is given into a pan and coated in the usual way with a solution consisting of:

| | |
|---|---|
| Cellulose acetate phthalate | 10 g |
| Dye E 172 | 0.4 g |
| Titanium dioxide | 0.2 g |
| Ethyl alcohol (96%) | q.s. to 100 g |

### Example 6
A homogeneous mixture of the following ingredients:

| | |
|---|---|
| L-Carnitine | 300 g |
| Lecithin | 500 g |
| Shellac | 80 g |
| Sorbitol | 1500 g |
| Silicon dioxide | 45 g |
| Magnesium stearate | 20 g |
| Saccharose | 200 g |
| Maize starch | 350 g |
| Ascorbic acid | 3 g |

is prepared by mixing L-carnitine and lecithin. The mixture is homogenized and pressed, and then milled and sieved in order to obtain homogeneous particle size. The other ingredients (shellac, excipient and additives) are added to the mixture and mixed again.

The obtained mixture is pressed on a rotary machine with punches of 22 mm in diameter, in order to obtain tablets of 3 g each.

### Example 7
A homogeneous mixture of the following ingredients:

| | |
|---|---|
| L-Carnitine | 150 g |
| Lecithin | 500 g |
| Rosin | 30 g |
| Turpentine | 10 g |
| Sorbitol | 550 g |
| Silicon dioxide | 40 g |
| Magnesium stearate | 17 g |
| Saccharose | 100 g |
| Maize starch | 300 g |
| Ascorbic acid | 3 g |

is prepared according to the method described in example 1 in order to obtain tablets of 1.7 g each.

# EP 0 206 240 B1

## Example 8

The physical stability of the tablets of the present invention has been evaluated in comparison to tablets having the following composition:

| | |
|---|---|
| L-Carnitine | 150 g |
| Lecithin | 500 g |
| Sorbitol | 550 g |
| Silicon dioxide | 40 g |
| Magnesium stearate | 17 g |
| Saccharose | 100 g |
| Maize starch | 300 g |
| Ascorbic acid | 3 g |

The comparison tablets are different from the tablets of the examples 1 and 2 only by the fact that the comparison samples do not contain the stabilizer according to the present invention.

The tablets of the examples 1, 2 and 3 (below indicated as tablets 1, 2 and 3 respectively) and the above described comparison tablets (tablets C) were packed separately in blister packages according to one of the usual tablet packaging methods. The blister packages were stored at different temperatures inspected periodically for the general state of preservation and samples of tablets withdrawn to analyse the physical state and the degree of preservation of the ingredients.

Generally it has been observed that the degree of preservation of the ingredients was good while the physical state of the tablet remained unaltered. The results are reported in the following table.

### TABLE 1
Period of physical stability of the tablets packed in blister packages[a]
stored at different temperatures

| Tablets[b] | 50°C | 40°C | 30°C | Room temperature |
|---|---|---|---|---|
| 1 | 6 months | 2 years | 2 years and 5 months | 3 years and 4 months |
| 2 | 6 months | 2 years and 2 months | 2 years and 6 months | 3 years |
| 3 | 6 months | 2 years and 6 months | 3 years | 4 years |
| C | 3 weeks | 2 months | 4 months | 6 months |

(a) Every value is the average of three parallel experiments, each of them carried out with 5 tablets of each type packed in blister packages.

(b) The tablets 1, 2, 3 and C (comparison) are those described respectively in the examples 1, 2, 3 and the present example.

## Example 9

The effectiveness and the good compatibility of the solid pharmaceutical preparations of the present invention have been tested with different patients showing conditions of psycho-physical asthenia or in convalescence, always with good results.

The below clinical cases show the effectiveness and the compatibility of the chewable tablets according to the present invention.

7

Case 1

A 68 year old male patient affected with non-specific chronic bronchopneumopathy was hospitalized because of an acute relapse of bacterial origin.

At the time of his hospitalization the patient was feverish and showed the symptoms of a respiratory insufficiency of medium seriousness. The spirometric examination showed a $FEV_1\%$ CV ratio equal to 61, $P_{CO_2}$ was 52 mbar (39 mmHg), $P_{O_2}$ was 118,6 mbar (89 mmHg).

The hematological and hematochemical tests were all normal, except for a slight leukocytosis (8500/$mm^3$) and a moderate increase of VES.

A therapy with theophylline derivatives by intravenous route, amoxycillin and with bronchodilators and secretolytics by inhalation was begun. After 7 days the patient did not show any respiratory symptoms but he showed notable signs of asthenia, anorexy and sleep-disturbances. A treatment with chewable tablets containing 150 mg of L-carnitine and 500 mg of pure soybean lecithin at the dosage of 6 tablets/day (tablets of example 3) was begun. The subjective condition of the patient was evaluated by the stress-test on a bicycle at 100 W.

The exercise was performed every 2 days for the whole period of the treatment, that is 21 days.

Starting from the 4th-5th day, an ascending improvement of the subjective symptomatology of the patient as well as an improved performance in the stress-test (expressed as an increase of time, from 1.8 minutes during the first day to 8.6 minutes during the 21st day) have been observed.

These observations show an improvement in the neuromuscular performance caused by the treatment, together with a good compatibility.

Case 2

A 59 year old female patient was hospitalized for a condition of severe asthenia after a long period of disease (specific pleurisy). Said condition had been lasting for more than two months, in spite of the fact that the patient stayed in bed and was treated with polyvitamins.

A treatment with chewable tablets containing 150 mg of L-carnitine and 500 mg of soybean lecithin, at the dose of 6 tablets/day (tablets of example 1) for a period of 30 days was begun. The body weight, the arterial pressure, the muscular strength (by a dynamometer) as well as the urinary elimination of the 17-ketosteroids were evaluated.

The evaluation of the subjective symptoms of asthenia, feelings of weariness, mental tiredness and sleep-disturbances was performed according to a scale of scores. There was observed a remarkable increase of appetite and an improvement of physical and psychic performance, together with an increase of 2.4 kg body weight.

The use of dynamometer showed an increase of the muscular strength of 32%. The treatment was tolerated very well.

Case 3

A 28 year old male patient was hospitalized because of a fracture of the right internal malleolus after a skiing accident. After surgical adjustment and motoric rehabilitation, the subject was treated for 4 weeks with an association of 150 mg of L-carnitine and 500 mg of soybean lecithin in the form of chewable tablets at the dose of 10 tablets/day (tablets of example 3).

The subject was in bed and examined twice a week in order to evaluate his physical performance. For this purpose he was subjected to a sub-maximum stress-test on an ergometric bicycle. Blood samples were withdrawn for the analyses of lactic acid.

At the end of the fourth week the subject's performance in the stress test had increased from 9 minutes to 32 minutes.

The examination of lactic acid after the stress-test showed a starting value of 10.6 mmol/l and a value of 5.9 mmol/l after 4 weeks of treatment.

No symptoms of intolerance of the therapy were observed.

Case 4

A 26 year old male sportman had been practising cycling at agonistic level for several years. His training was very regular, at present he is in good general health.

A month ago he complained about tiredness perhaps in consequence of the mediocre results obtained during the season. He was treated by the team physician with chewable tablets containing 150 mg of L-carnitine and 500 mg of soybean lecithin (tablets of example 2), at the dosage of 4 tablets every 6 hours for a period of 6 weeks.

The subject presented himself regularly for the medical check once a week, his general state of health was evaluated and a sub-maximum stress-test on an ergometic bicycle was performed. Already after two weeks of treatment the subject no longer complained about tiredness, he felt in very good form and noted an increase of appetite.

The stress-test showed an increase of the muscular resistance, the longest time observed increased from 37 minutes before the treatment to 47 minutes after the 3rd week and to 64 minutes at the 6th week. The subject did not feel any undesirable effect from the treatment.

**Claims for the Contracting States: BE CH DE FR GB IT LI LU NL SE**

1. A solid pharmaceutical composition for oral use consisting of carnitine or a derivative thereof, lecithin and usual excipients and additives, characterized by the fact that it contains also an amount comprised between 1 and 60% weight by weight, in respect of the weight of carnitine plus lecithin, of a substance selected among shellac, rosin, turpentine, cellulose acetate phthalate, polyacrylamide resin or their mixtures.

2. A solid pharmaceutical composition according to claim 1 in the form of a chewable tablet.

3. A solid pharmaceutical composition according to one or both of the claims 1—2, coated with a substance selected among shellac, rosin, turpentine, cellulose acetate phthalate, polyacrylamide resin or their mixtures.

4. A solid pharmaceutical composition according to one or more of the claims 1—3, consisting of carnitine or a derivative thereof (5—15% w/w), lecithin (10—40% w/w), a substance selected among shellac, rosin, turpentine, cellulose acetate phthalate, polyacrylamide resin or their mixtures (1—5% w/w), the rest to 100% w/w being constituted by pharmacologically acceptable excipients and additives suitable for the preparation of solid pharmaceutical compositions for oral use.

5. A solid pharmaceutical composition according to one or more of the claims 1—4 in which carnitine or a derivative thereof is L-carnitine, D,L-carnitine, L-acetylcarnitine, D,L-acetylcarnitine or a pharmacologically acceptable salt thereof.

6. A solid pharmaceutical composition according to one or more of the claims 1—5 in which carnitine is L-carnitine.

7. A solid pharmaceutical composition according to one or more of the claims 1—6 in which lecithin is soybean lecithin.

**Claims for the Contracting State: AT**

1. A method for the stabilization of a solid pharmaceutical composition for oral use, said composition consisting of carnitine or a derivative thereof, lecithin and usual excipient and additives, characterized by combining said composition with a substance selected among shellac, rosin, turpentine, cellulose acetate phthalate, polacrylamide resin or their mixtures in an amount comprised between 1 and 60% weight by weight, based on the weight of carnitine plus lecithin.

2. A method according to claim 1 wherein said solid pharmaceutical composition is in the form of a chewable tablet.

3. A method according to one or both of the claims 1—2, wherein said solid pharmaceutical composition is coated with a substance selected among shellac, rosin, turpentine, cellulose acetate phthalate, polyacrylamide resin or their mixtures.

4. A method according to one or more of the claims 1—3, resulting in a solid pharmaceutical composition consisting of carnitine or a derivative thereof (5—15% w/w), lecithin (10—40% w/w), a substance selected among shellac, rosin, turpentine, cellulose acetate phthalate, polyacrylamide resin or their mixtures (1—5% w/w), the rest to 100% w/w being constituted by pharmacologically acceptable excipients and additives suitable for the preparation of solid pharmaceutical compositions for oral use.

5. A method according to one or more of the claims 1—4, in which said solid pharmaceutical composition contains, as carnitine or derivative thereof, L-carnitine, D,L-carnitine, L-acetylcarnitine, D,L-acetylcarnitine or a pharmacologically acceptable salt thereof.

6. A method according to one or more of the claims 1—5, in which said solid pharmaceutical composition contains, as carnitine, L-carnitine.

7. A method according to one or more of the claims 1—6, in which said solid pharmaceutical composition contains, as lecithin, soybean lecithin.

**Patentansprüche für die Vertragsstaaten: BE CH DE FR GB IT LI LU NL SE**

1. Feste pharmazeutische Zusammensetzung zur oralen Verwendung, bestehend aus Carnitin oder einem Derivat desselben, Lecithin und üblichen Exzipientien und Zusätzen, dadurch gekennzeichnet, daß sie ferner eine Menge zwischen 1 und 60% Gew./Gew., bezogen auf das Gewicht von Carnitin plus Lecithin, einer Substanz enthält, die aus Schellack, Kolophonium, Terpentin, Celluloseacetatphthalat, Polyacrylamidharz oder deren Mischungen ausgewählt ist.

2. Feste pharmazeutische Zusammensetzung nach Anspruch 1 in Form einer Kautablette.

3. Feste pharmazeutische Zusammensetzung nach einem oder beiden der Ansprüche 1 bis 2, die mit einer Substanz überzogen ist, die aus Schellack, Kolophonium, Terpentin, Celluloseacetatphthalat, Polyacrylamidharz oder deren Mischungen ausgewählt ist.

4. Feste pharmazeutische Zusammensetzung nach einem oder mehreren der Ansprüche 1 bis 3, bestehend aus Carnitin oder einem Derivat desselben (5—15% Gew./Gew.), Lecithin (10—40% Gew./Gew.), einer aus Schellack, Kolophonium, Terpentin, Celluloseacetatphthalat, Polyacrylamidharz oder deren Mischungen ausgewählten Substanz (1—5% Gew./Gew.), wobei der Rest auf 100% Gew./Gew. aus pharmakologisch annehmbaren Exzipientien und Zusätzen besteht, die zur Herstellung fester pharmazeutischer Zusammensetzungen zur oralen Verwendung geeignet sind.

5. Feste pharmazeutische Zusammensetzung nach einem oder mehreren der Ansprüche 1 bis 4, in welcher Carnitin oder ein Derivat desselben L-Carnitin, D,L-Carnitin, L-Acetylcarnitin, D,L-Acetylcarnitin oder ein pharmakologisch annehmbares Salz derselben ist.

6. Feste pharmazeutische Zusammensetzung nach einem oder mehreren der Ansprüche 1 bis 5, in welcher das Carnitin L-Carnitin ist.

7. Feste pharmazeutische Zusammensetzung nach einem oder mehreren der Ansprüche 1 bis 6, in welcher das Lecithin Sojabohnen-Lecithin ist.

**Patentansprüche für den Vertragsstaat: AT**

1. Verfahren zum Stabilisieren einer festen pharmazeutischen Zusammensetzung zur oralen Verwendung, bestehend aus Carnitin oder einem Derivat desselben, Lecithin und üblichen Exzipientien und Zusätzen, dadurch gekennzeichnet, daß die Zusammensetzung mit einer Substanz, die aus Schellack, Kolophonium, Terpentin, Celluloseacetatphthalat, Polyacrylamidharz oder deren Mischungen ausgewählt ist, in einer Menge zwischen 1 und 60% Gew./Gew., bezogen auf das Gewicht von Carnitin plus Lecithin, kombiniert wird.

2. Verfahren nach Anspruch 1, worin die feste pharmazeutische Zusammensetzung in Form einer Kautablette vorliegt.

3. Verfahren nach einem oder beiden der Ansprüche 1 bis 2, worin die feste pharmazeutische Zusammensetzung mit einer aus Schellack, Kolophonium, Terpentin, Celluloseacetatphthalat, Polyacrylamidharz oder deren Mischungen ausgewählten Substanz überzogen wird.

4. Verfahren nach einem oder mehreren der Ansprüche 1 bis 3, das zu einer festen pharmazeutischen Zusammensetzung führt, welche aus Carnitin oder einem Derivat desselben (5—15% Gew./Gew.), Lecithin (10—40% Gew./Gew.), einer aus Schellack, Kolophonium, Terpentin, Celluloseacetatphthalat, Polyacrylamidharz oder deren Mischungen ausgewählten Substanz (1—5% Gew./Gew.) besteht, wobei der Rest auf 100% Gew./Gew. aus pharmakologisch annehmbaren Exzipientien und Zusätzen besteht, die zur Herstellung fester pharmazeutischer Zusammensetzungen zur oralen Verwendung geeignet sind.

5. Verfahren nach einem oder mehreren der Ansprüche 1 bis 4, in welchem die feste pharmazeutische Zusammensetzung als Carnitin oder dessen Derivat L-Carnitin, D,L-Carnitin, L-Acetylcarnitin, D,L-Acetylcarnitin oder ein pharmakologisch annehmbares Salz derselben enthält.

6. Verfahren nach einem oder mehreren der Ansprüche 1 bis 5, in welchem die feste pharmazeutische Zusammensetzung als Carnitin L-Carnitin enthält.

7. Verfahren nach einem oder mehreren der Ansprüche 1 bis 6, in welchem die feste pharmazeutische Zusammensetzung als Lecithin Sojabohnen-Lecithin enthält.

**Revendications pour les Etats contractants: BE CH DE FR GB IT LI LU NL SE**

1. Composition pharmaceutique solide pour administration orale, se composant de carnitine ou de l'un de ses dérivés, de lécithine et d'excipients et additifs usuels, caractérisée en ce qu'elle contient aussi une proportion, comprise entre 1 et 60% en poids par rapport au poids de carnitine plus lécithine, d'une substance choisie parmi la gomme-laque, la colophane, la térébenthine, l'acétophtalate de cellulose, la résine polyacrylamide ou leurs mélanges.

2. Composition pharmaceutique solide selon la revendication 1, sous la forme d'une tablette à mâcher.

3. Composition pharmaceutique solide selon la revendication 1 ou 2, revêtue d'une substance choisie parmi la gomme-laque, la colophane, la térébenthine, l'acétophtalate de cellulose, la résine polyacrylamide ou leurs mélanges.

4. Composition pharmaceutique solide selon l'une quelconque des revendications 1 à 3, se composant de carnitine ou d'un dérivé de celle-ci (5—15% en poids), de lécithine (10—40% en poids), d'une substance choisie parmi la gomme-laque, la colophane, la térébenthine, l'acétophtalate de cellulose, la résine polyacrylamide ou leurs mélanges (1—5% en poids), le reste à 100% en poids étant constitué par des excipients et additifs acceptables sur le plan pharmacologique, convenant pour la préparation de compositions pharmaceutiques solides pour administration orale.

5. Composition pharmaceutique solide selon l'une quelconque des revendications 1 à 4, dans laquelle la carnitine ou son dérivé est la L-carnitine, la D,L-carnitine, la L-acétylcarnitine, la D,L-acétylcarnitine ou un sel de celles-ci acceptable sur le plan pharmacologique.

6. Composition pharmaceutique solide selon l'une quelconque des revendications 1 à 5, dans laquelle la carnitine est de la L-carnitine.

7. Composition pharmaceutique solide selon l'une quelconque des revendications 1 à 6, dans laquelle la lécithine est de la lécithine de soja.

**Revendications pour l'Etat contractant: AT**

1. Procédé de stabilisation d'une composition pharmaceutique solide pour administration orale, cette composition se composant de carnitine ou de l'un de ses dérivés, de lécithine et d'excipients et additifs usuels, caractérisé en ce qu'on combine ladite composition avec une substance choisie parmie la gomme-laque, la colophane, la térébenthine, l'acétophtalate de cellulose, la résine polyacrylamide ou leurs mélanges dans une proportion comprise entre 1 et 60% en poids sur la base du poids de carnitine plus lécithine.

2. Procédé selon la revendication 1, dans lequel ladite composition pharmaceutique solide est sous la forme d'une tablette à mâcher.

3. Procédé selon la revendication 1 ou 2, dans lequel ladite composition pharmaceutique solide est revêtue d'une substance choisie parmi la gomme-laque, la colophane, la térébenthine, l'acétophtalate de cellulose, la résine polyacrylamide ou leurs mélanges.

4. Procédé selon l'une quelconque des revendications 1 à 3, donnant une composition pharmaceutique solide se composant de carnitine ou d'un dérivé de celle-ci (5—15% en poids), de lécithine (10—40% en poids), d'une substance choisie parmi la gomme-laque, la colophane, la térébenthine, l'acétophtalate de cellulose, la résine polyacrylamide ou leurs mélanges (1—5% en poids), le reste à 100% en poids étant constitué par des excipients et additifs acceptables sur le plan pharmacologique, convenant pour la préparation de compositions pharmaceutiques solides pour administration orale.

5. Procédé selon l'une quelconque des revendications 1 à 4, dans lequel ladite composition pharmaceutique solide contient, en tant que carnitine ou de dérivé de celle-ci, de la L-carnitine, de la D,L-carnitine, de la L-acètylcarnitine, de la D,L-acétylcarnitine ou un sel de celles-ci acceptable sur le plan pharmacologique.

6. Procédé selon l'une quelconque des revendications 1 à 5, dans lequel ladite composition pharmaceutique solide contient, en tant que carnitine, de la L-carnitine.

7. Procédé selon l'une quelconque des revendications 1 à 6, dans lequel ladite composition pharmaceutique solide contient, en tant que lécithine, de la lécithine de soja.